# EUROPEAN PATENT APPLICATION

(11) **EP 4 142 331 A1**
(43) Date of publication of application: **01.03.2023**
(21) Application number: 22190281.0
(22) Date of filing: 12.08.2022
(51) Int. Cl.: H04W 12/33, H04W 12/50, H04L 9/40, G06F 21/44

(54) **SYSTEM, DEVICE AND METHOD FOR MULTI-DEVICE AUTHENTICATION**

(30) Priority: 26.08.2021 US 202117446005
(71) Applicant: NXP B.V., 5656 AG Eindhoven (NL)
(72) Inventor: Meijer, Rinze Ida Mechtildis Peter, 5656 AG Eindhoven (NL); Rajan Kesavelu Shekar, Pramod, 5656 AG Eindhoven (NL)
(74) Representative: Miles, John Richard

(57) **Abstract**

One example discloses a resource management device, including: an input interface configured to receive a first set of sensor readings corresponding to a user and a second set of sensor readings corresponding to a set of resources; a controller configured to match the user, based on the first set of sensor readings, to a specific resource within the set of resources, based on the second set of sensor readings; wherein the controller is configured to authenticate that the first set of sensor readings originated from the user before the match.

## Description

The present specification relates to systems, methods, apparatuses, devices, articles of manufacture and instructions for closed-loop operations between a set of resource management devices.

### SUMMARY

According to an example embodiment, a resource management device, comprising: an input interface configured to receive a first set of sensor readings corresponding to a user and a second set of sensor readings corresponding to a set of resources; a controller configured to match the user, based on the first set of sensor readings, to a specific resource within the set of resources, based on the second set of sensor readings; wherein the controller is configured to authenticate that the first set of sensor readings originated from the user before the match.

In another example embodiment, the first set of sensor readings correspond to a set of glucose readings; the set of resources include insulin; and the second set of sensor readings correspond to an attribute of the insulin.

In another example embodiment, the controller is configured to authenticate the set of resources correspond to the second set of sensor readings before the match.

In another example embodiment, the input interface is a near-field wireless communications interface.

In another example embodiment, the first set of sensor readings are generated from the user via a patch coupled to the user; and the near-field interface is enabled only when the user is holding or touching the specific resource.

In another example embodiment, the patch is a glucose sensor patch; and the specific resource is an insulin pen.

In another example embodiment, the first set of sensor readings are generated from the user via a bio-identity sensor coupled to identify the user; and the near-field interface is enabled only when the user is touching the specific resource.

In another example embodiment, the controller is configured to wake-up the resource management device from a low power state in response to a trigger event from the user.

In another example embodiment, the specific resource is an insulin pen; and the trigger event corresponds to the user removing a pen cap from the insulin pen.

In another example embodiment, further comprising an authentication unit configured to enable the controller to authenticate by verifying at least one of: a unique identifier (ID), a manufacturing certificate, a remaining amount of the specific resource, an expiration of the specific resource, an environmental history of the specific resource, and/or a type of the specific resource.

In another example embodiment, the specific resource is an insulin pen; and further comprising an authentication unit configured to enable the controller to authenticate by verifying at least one of: a unique identifier (ID) of the insulin pen, a manufacturing certificate for the insulin pen, a remaining amount of insulin in the insulin pen, an expiration date of the insulin in the insulin pen, an environmental history of the insulin in the insulin pen, and/or a type of insulin in the insulin pen.

In another example embodiment, the first set of sensor readings correspond to a set of glucose readings from a patch coupled to the user; and further comprising an authentication unit configured to enable the controller to authenticate by verifying at least one of: a unique identifier (ID) of the patch, a manufacturing certificate for the patch, a glucose measurement log in the patch, and/or an insulin dosage history in the patch.

In another example embodiment, the first set of sensor readings correspond to a set of glucose readings from a patch coupled to the user; and further comprising an alerting unit configured to enable the controller to alert the user when the user needs another insulin injection within a predefined time window.

In another example embodiment, the alert is a haptic alert that vibrates the user.

In another example embodiment, the specific resource is insulin; the first set of sensor readings correspond to a set of glucose readings from a patch coupled to the user; and further comprising an alerting unit configured to enable the controller to alert the user when the specific resource contains an insufficient amount or incorrect type of the insulin needed for injection by the user.

In another example embodiment, the specific resource is a first specific resource and is not available to the user; the controller is configured to match the user, based on the first set of sensor readings, to a second specific resource within the set of resources, based on the second set of sensor readings; and the controller is configured to direct the user to a location of the second specific resource.

In another example embodiment, the first specific resource is a first insulin pen, and the second specific resource is a second insulin pen.

In another example embodiment, the user is at least one of: a patient, a worker, a robot, or a vehicle.

In another example embodiment, the set of resources include at least one of: a medicine, insulin, a drug, a food, a vaccine, a treatment, a service, an assembly operation, a repair operation, an inspection, a maintenance operation, an access permission, computing resources, power resources, a reservation, an instrument use, a machine use, or vehicle services.

In another example embodiment, the specific resource is an insulin pen; and the controller configured to command the insulin pen to inject the insulin into the user.

In another example embodiment, the specific resource is an injection pen; and the trigger event corresponds to the user removing a pen cap from the injection pen.

According to an example embodiment, a method of distributing instructions, stored on a non-transitory, tangible computer readable storage medium, for configuring a resource management device: wherein the device includes an input interface and a controller; and wherein the instructions include, configuring the input interface to receive a first set of sensor readings corresponding to a user and a second set of sensor readings corresponding to a set of resources; configuring the controller to match the user, based on the first set of sensor readings, to a specific resource within the set of resources, based on the second set of sensor readings; and wherein the controller is configured to authenticate the user and the specific resource before the match.

The above discussion is not intended to represent every example embodiment or every implementation within the scope of the current or future Claim sets. The Figures and Detailed Description that follow also exemplify various example embodiments.

Various example embodiments may be more completely understood in consideration of the following Detailed Description in connection with the accompanying Drawings.

### BRIEF DESCRIPTION OF THE DRAWINGS

Figure 1 represents an example closed-loop system between a set of resource management devices.
Figure 2 represents an example set of resource management devices.
Figure 3 represents an example authentication process for one or more on-body and off-body devices.
Figure 4 represents a first example closed-loop system between the set of resource management devices.
Figure 5 represents a second example closed-loop system between the set of resource management devices.

While the disclosure is amenable to various modifications and alternative forms, specifics thereof have been shown by way of example in the drawings and will be described in detail. It should be understood, however, that other embodiments, beyond the particular embodiments described, are possible as well. All modifications, equivalents, and alternative embodiments falling within the spirit and scope of the appended claims are covered as well.

### DETAILED DESCRIPTION

With a growing Healthcare Internet-of-Things (IoT), Continuous Glucose Monitoring (patch) systems are taking hold. Glucose systems for diabetes care are designed to keep glucose levels within target range using a closed-loop approach that does not require direct patient input. They include smart glucose sensor patches and smart insulin pens connected in real-time to using a smart hub device (e.g. a smartphone).

The glucose sensor patches are body-worn devices that deliver real time information to help people proactively manage their diabetes. This system measures a person's glucose level in real time by placing a tiny electrode sensor under the skin. The sensor then can communicate with a transmitter to send the information of the glucose levels to a connected device such as mobile app on any smartphone.

While durable smart insulin pens are reusable and accept refillable insulin cartridges, use of disposable pens is growing. With the growing popularity of disposable insulin pens, diabetes patients using such pens need to be even more vigilant prior to any insulin injection. A non-exhaustive list of several of such safety items patients need to check, include: that genuine devices are used; the amount of insulin to be injected (injection of an improper amount may cause a high-risk of death); the remaining insulin in the pen, and whether there is enough for the required injection; the temperature of the insulin is within a given temperature range prior to injection; the insulin inside the pen has not passed its expiration date; and that injection dose logs kept (especially important for medical personnel that injects insulin into more than a single diabetes patient).

Unlike on-body insulin pumps or single dedicated insulin pen, a patient may have access to many different off-body disposable smart insulin pens which makes an automatic closed-loop operation potentially dangerous. Also with so many disposable pens, the patient is presented with a difficult task of ensuring the insulin pen they currently plan to use found is a safe pen to inject insulin with.

Furthermore, today's smart insulin pens require the presence of a third device (i.e. a so-called hub-device (e.g. patch reader device or smart phone)) to guide the insulin injection process. Without such hub device, a patient is not capable to determine the amount of insulin to be injected with a particular pen.

Now discussed are systems, devices, and methods for closed-loop operations between multiple resource management devices, which are particularly useful for protecting users (e.g. patients) in medical (e.g. insulin delivery) or other high-risk applications.

Some example applications include a closed-loop operation between a glucose sensor patch and a smart insulin pen. These applications:
- enable closed-loop 1:1 wireless communication between sensor patch and the smart insulin pen;
- remove an explicit requirement for a hub device, to determine actual glucose reading and guidance of patient on dose setting. However, in some example embodiments a "reader device" (i.e. not a "hub device") for reading dose logs/glucose trends or any graphical means to plotting such information can be used (e.g. implemented by a smartphone).
- offer private, low-latency and low-power communication utilizing body-coupled near-field communication;
- guide diabetes patients (or medical personnel) on insulin dose setting based on actual glucose reading of the patient. In some example embodiments the guiding would require some sort of "reader device" and/or 'display function". Note that some disposable patches and/or pens may not have such a display due to cost.
- enable automated dose setting at the insulin pen based on actual glucose sensor patch reading;
- enable a patient to still perform further correction for bolus insulin injections (e.g. to compensate for diner etc.);
- automatically check on insulin related details such as expiration date, actual temperature of the insulin, and whether insulin has been exposed to temperature excursions outside the acceptable temperature window;
- provide an authentication means for connecting given (i.e. one or more) smart insulin pen(s) to given (i.e. one or more) glucose patches(s) worn by a patient;
- provide authorization for each of the given pens and patches for validating their genuineness and the insulin used; and
- to connect one or more smart insulin pens to a given diabetes patient that wears a given sensor device, which may also be used to block insulin injection when the pen is used on a non-authenticated patient.

However other example embodiments using closed-loop operations between a set of resource management devices include matching a set of users to a set of resources. For example these applications may include administration of a variety of medicines or medical services to a patient, delivery of a variety of resources to a variety of users, logging an operator's interaction with a machine, and so on.

The term "user", in addition to its ordinary meaning, is herein broadly defined to also include: a patient, a worker, a robot, or a vehicle.

The term "resource", in addition to its ordinary meaning, is herein broadly defined to also include: a medicine, insulin, a drug, a food, a vaccine, a treatment, a service, a maintenance procedure, an assembly operation, a repair operation, an inspection, an access permission, computing resources, power resources, a reservation, an instrument use, a machine use, or vehicle services.

The following example Figures discuss in detail a closed-loop operation between a glucose sensor patch and a smart insulin pen, however, the Claims that follow read-on many other closed-loop operations between a set of resource management devices enabled by this specification.

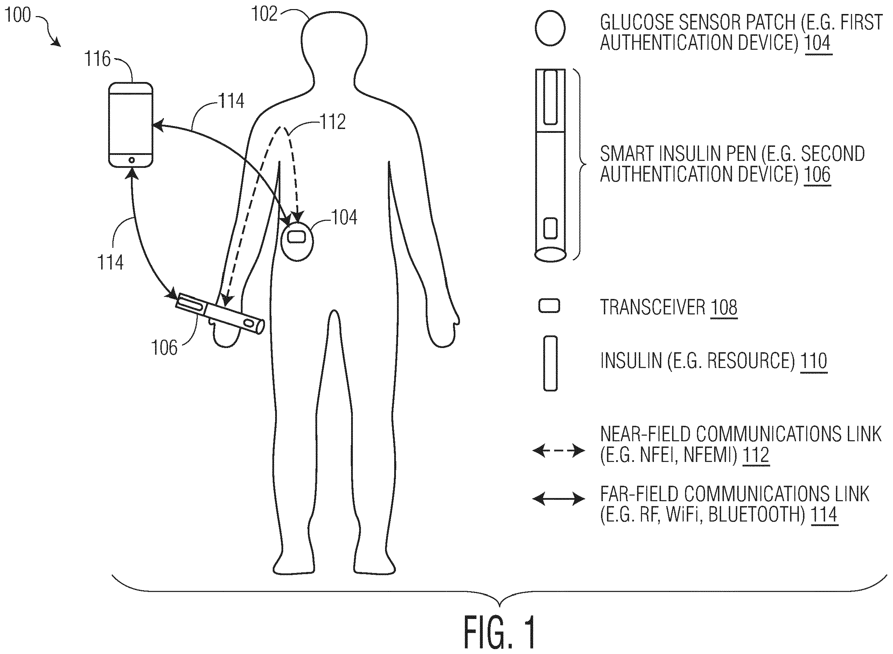

Figure 1 represents an example closed-loop operation 100 between a set of resource management devices for delivery of insulin to a patient. The closed-loop operation 100 is not limited to insulin or other medicines, but has application to various other applications that require verification and/or tracking, as will be further discussed below.

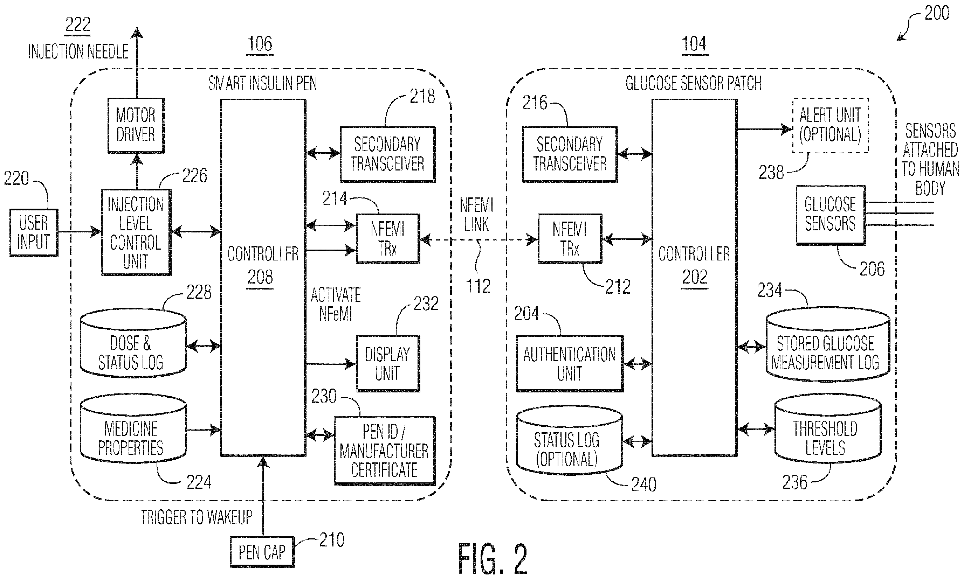

The example closed-loop operation 100 shows a patient 102 wearing a glucose sensor patch (e.g. first resource management device, CGM (Continuous Glucose Monitor), etc.) 104 and holding a smart insulin pen (e.g. second resource management device) 106. The patch 104 collects actual glucose readings from the patient 102 (i.e. a diabetes patient) and the pen 106 injects insulin. Either or both the patch 104 and the pen 106 can include an authentication device or module (e.g. hardware, software or a combination thereof such as shown in Figures 2-5).

Both the patch 104 and pen 106 include a transceiver 108. The transceiver 108 includes a near-field transceiver that hosts a near-field communication link 112 (e.g. NFEMI, NFEI or NFMI) between the patch 104 and pen 106. Both the patch 104 and pen 106 are herein defined as on-body devices since they are attached to or held by the patient 102. The near-field communication link 112 can be enabled by a near-field electromagnetic induction (NFEMI) device, a near-field electric induction (NFEI) device, or a near-field magnetic induction (NFMI) device.

In some example embodiments, the transceiver 108 may optionally include a far-field transceiver that hosts a far-field communication link 114 (e.g. RF, WiFi, Bluetooth, BLE, etc.) between the patch 104 and pen 106 themselves, and/or an off-body device (e.g. a smartphone, a server, a cloud connection, etc.) 116. The pen 106 includes an insulin cartridge (e.g. resource) 110.

Figure 2 represents an example 200 set of resource management devices. The example 200 shows the glucose sensor patch (e.g. a first resource management device) 104 and the smart insulin pen (e.g. second resource management device) 106. The patch 104 includes a controller 202, an authentication unit 204, and a glucose sensor 206. The pen 106 includes a controller 208 and a pen cap 210. The near-field communication link 112 between the near-field (e.g. NFEMI) transceivers 212, 214 in the patch 104 and pen 106 is shown. A potential for the far-field communication link 114 is enabled by secondary transceivers 216, 218 in the patch 104 and pen 106 is also shown.

In some example embodiments, removal of the pen cap 210 is defined by the controller 208 as a trigger to wake-up the pen 106. By triggering this input, the controller 208 gets activated and consequently the rest of the pen's 106 electronics as well. The NFEMI radio transceiver 214 gets enabled to initiate setting-up of the near-field radio link 112. In some example embodiments, there are alternative wake-up events possible (e.g. turning of the rotary knob, or pushing a button).

Besides the automatic dose setting, the patient 102 is still capable to modify the insulin dose setting with a user input 220. This may be needed to account for a bolus settings, prior to the patient 102 starting a meal. The pen 106 includes a pen needle 222 for injecting insulin into the patient 102. After doublechecking the correct insulin dose setting, the patient 102 can press a button/plunger to inject the insulin.

The pen 106 includes a medicine properties memory 224. This memory 224 stores a number of properties about the insulin. Example of such properties are: a type of insulin and an expiration date, temperature history of the insulin, and a number of times the temperature limits were exceeded.

The NFEMI transceiver 214 in the pen 106 is configured to set-up a near-field wireless communication channel with the patch 104 via the patient's 102 body so that the medicine properties can be provided to the patch 104, and a calculated dose setting is received from the patch 104.

The controller 208 controls the complete electronics in the smart insulin pen 106. It activates the entire system when the wake up trigger even is received. It takes care of the sequencing associated to the proposed NFEMI communication and takes care of data packetization and depacketization process. It further takes care of setting the receive dose setting at the Injection Level Control Unit 226. Finally, it monitors what dose has eventually been injected and stores this together with a time-stamp in a Dosage & Status Log memory 228.

The pen ID/manufacture certificate 230 stores a unique pen ID and a certificate provided by the manufacturer to ensure a genuineness of the product. Certificate validation is performed to check if the product is from a genuine manufacturing company. The display unit 232 is configured to display the relevant information to the patient 102. This information may include, among others, a calculated dose level as received from the patch 104, and a remaining dosage levels on the insulin pen.

In some embodiments, the display unit 232 is omitted from the smart insulin pen (e.g. due to cost reasons). The display function would then be required to be implemented by an external reader device (which is connected to a pen and/or patch via far-field wireless communication).

The injection level control unit 226 and connected motor driver sets an insulin dose to be injected based on the received details from the patch 104. The patient 102 can modify this setting as from the user input 220. The motor driver performs the actual insulin injection after user input. The dosage & status log 228 stores the injected insulin dosage levels in a memory portion of the smart insulin pen. The dosage log 228 is updated every time when the patient 102 injects the insulin, i.e. a time-stamped dose is recorded. The dosage log 228 can provide status regarding the remaining amount of insulin to the patient 102. In some example embodiments the dosage log 228 can use the far-field communication link 114 to share the dosage log 228 information with other smart insulin pens (not shown) that the patient 102 may use. The dosage log 228 can use the far-field communication link 114 to share the dosage log 228 information with other reader devices (e.g. smart phone) as well.

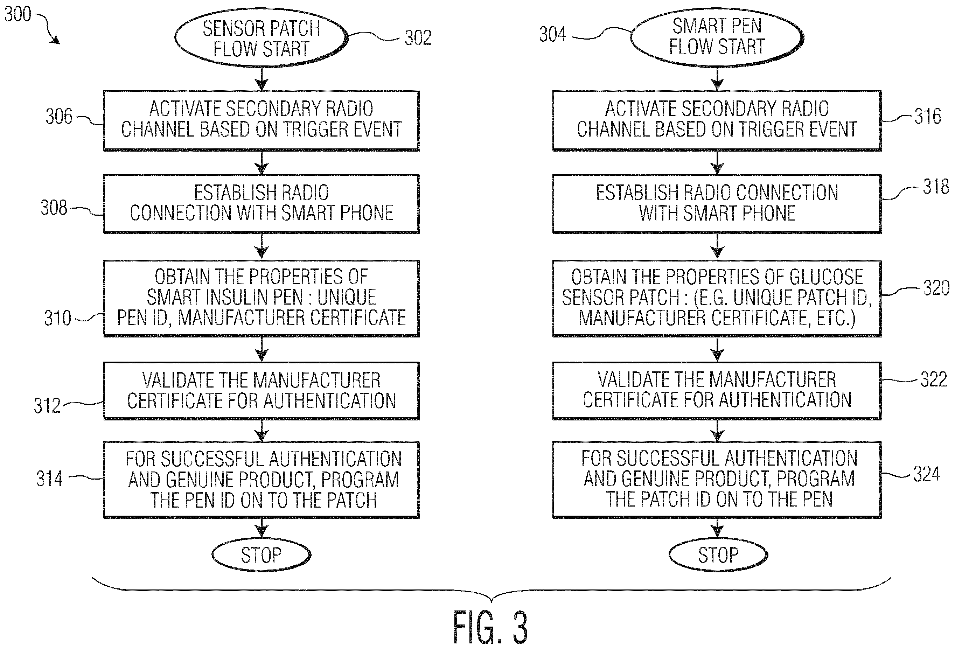

The secondary transceivers 216, 218 provide an additional radio channel (i.e. far-field communication link 114), to connect the smart insulin pen 106 to other off-body devices (e.g. other smart pens, a smartphone, a network access point, etc.). In some example embodiments connectivity may be NFC or BLE. One purpose of this additional channel is to provide the patient 102 with the possibility to look at their dose logs etc. on their other smart devices and/or on their computer. Alternative usage is that this channel can be used by a doctor's office, to read out various data stored in the pen 106 or patch 104. This radio channel can also be used for supporting the authentication process between patch 104 and pen (see Figure 3).

The patch 104 is connected to the glucose sensor 206 that determine the glucose level of the patient 102. In some example embodiments this sensing is done continuously (e.g. a new glucose value every minute), and the data is provided to the controller 202 in the patch 104. A stored glucose measurement log 234 stores the glucose information sensed through the glucose sensor 206. The stored data is time-stamped. Threshold levels 236 store a minimum and maximum acceptable levels of glucose to be present on the human body.

The NFEMI transceiver 212 sets-up a near-field wireless communication channel with the smart insulin pen 106 via the human body. In some example embodiments, the medicine properties are received from the pen 106, and the calculated dose setting is provided to the pen 106.

The controller 202 is the main processing unit and performs multiple individual processing tasks, such as: continuously, processing the sensed glucose information from the glucose sensor 206, storing the processed data into the stored glucose measurement log 234; continuously monitoring the sensed glucose information to be within the acceptable limits of threshold levels from the latest sensed data; triggering an alert unit to alert the patient 102 whenever the sensed glucose information is not within the predefined acceptable limits; receiving the medicine properties from the pen 106 through the near-field communication link 112 and calculating the amount of Insulin to be injected by considering the latest sensed glucose level of the patient 102; and transmitting calculated details of insulin to be injected to the pen 106 through the near-field communication link 112.

The authentication unit 204 stores the patch 104 ID, which is a unique code for the patch 104. This patch 104 ID can be shared with one or more insulin pens. In some example embodiments, the authenticated patch ID code is also stored in the pen 106, which is also checked as part of the authentication process. Similarly, the authenticated pen IDs of pens 106 are stored in the patch 104.

The patch 104 may include an alert unit 238 (optional) to alert the patient 102 whenever the monitored glucose information exceeds the threshold levels. In some example embodiments the alert is a haptic trigger (i.e. vibration, buzzer, tingling, light etc.) that catches the patient's 102 attention even when not looking at the patch 104. For example, the patch 104 may often be worn beneath clothes or at body locations where the patient can't see it easily (e.g. back-side of the arm) and looking at the patch 104 to become aware of an alarm may not be preferrable to a patient. Alarms may also be communicates to reader devices via the far-field communication link 114. These reader devices may be another way that patients receive an alarm. Such reader devices could be a smart phone and/or smart watch. Having a haptic trigger however may omit a need for another alarm on the user/patient's smart phone or smart watch.

A status log 240 (optional) stores the calculated amount of insulin to be injected.

The secondary transceiver 216 provides an additional radio channel (e.g. far-field communication link 114), to connect the patch 104 to other off-body smart devices. This devices may be a smart phone or the like. In some example embodiments connectivity may be NFC or BLE. One purpose of this additional channel is to provide the patient 102 with a possibility to look at the glucose readings etc. on their smart device. Alternatively this channel can be used by doctor's office, to read out the data. This radio channel is also used for supporting the authentication process between patch 104 and pen 106 (see Figure 3).

While in the discussion above, just the patch 104 includes the authentication unit 204, in other example embodiments the pen 106 could also include another authentication units.

Figure 3 represents an example 300 authentication process for one or more on-body and off-body devices (e.g. the patch 104, pen 106, and smartphone 116). To begin, in case of an example first time use of a patch 104 or a smart insulin pen 106, the patient 102 needs to: validate a genuineness of the smart insulin pen 106 and store the pen's 106 ID(identifier) inside the patch 104; and validate a genuineness of the patch 104 and program the patch's 104 ID inside the smart insulin pen 106.

In various example embodiments, the smart insulin pen 106 and patch 104 can store multiple IDs for various on-body and off-body devices, including not only multiple patches 104 and multiple pens 106, but also any other devices that are to be authenticated.

In this example embodiment, the secondary transceivers 216, 218 (e.g. far-field communication link 114) in the patch 104 and pen 106 are used to enable the authorization process using the off-body device (e.g. smartphone) 116.

Flow 302 provides an example patch to pen authentication process, and flow 304 provides an example pen to patch authentication process. Each flow 302, 304 activates a secondary radio channel based on a trigger event in steps 306, 316. Then radio connection with the smart phone 116 is established in steps 308, 318.

In step 310 the patch 104 obtains the properties of the smart insulin pen 106 (e.g. a unique pen ID, a pen manufacturer certificate, an amount of glucose remaining, dosage log history, etc.). In step 320 the pen 106 obtains the properties of the patch 104 (e.g. a unique patch ID, a patch manufacturer certificate, glucose history, etc.).

In steps 312, 322 an authenticity check of the manufacturer certificates is obtained from the patch 104 and pen 106 are validated by the smartphone 116. Then in step 314, upon successful authentication, the patch 104 stores the pen ID, and in step 324 the pen 106 stores the patch ID.

A benefit of the authentication process is that it offers a solution to prevent use of non-genuine products - which can be harmful to the patient 102 or can cause damage to the manufacturer's brand. Authentication also offers a means to connect use of the glucose by the patient 102 for follow-up doctor's care.

Note, the order in which these instructions have been discussed does not limit the order in which other example embodiments implement the instructions unless otherwise specifically stated. Additionally, in some embodiments the instructions are implemented concurrently.

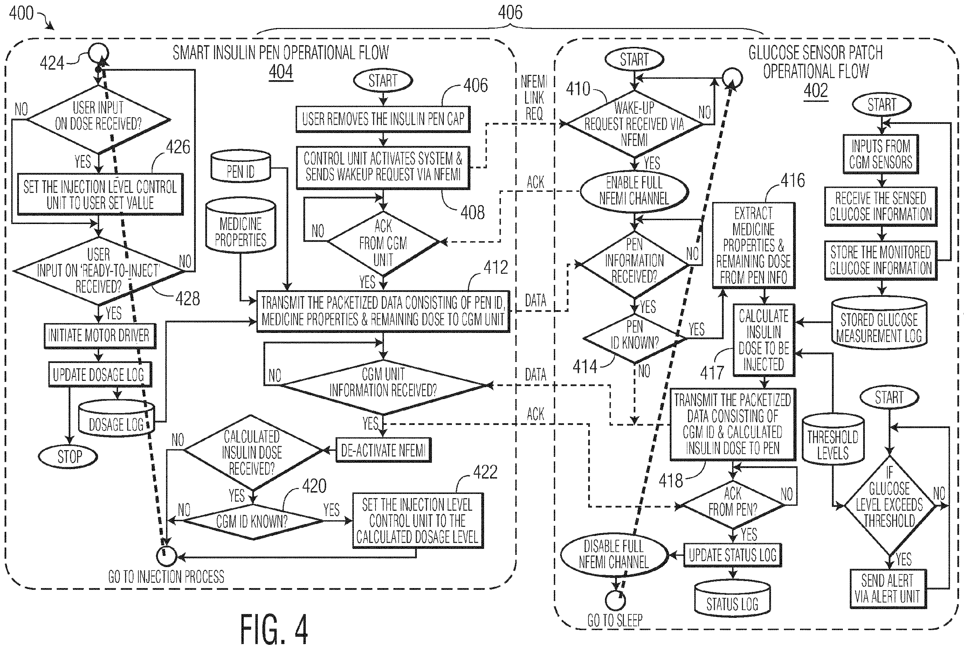

Figure 4 represents a first example 400 closed-loop operation 100 between the set of resource management devices (e.g. the patch 104 and the pen 106). The example 400 shows a glucose sensor patch 104 operational flow 402, a smart insulin pen 106 operational flow 404, and an authentication process 406 distributed between the patch 104 and the pen 106. To begin the patch 104 is worn by the patient 102 and actively collects the patient's 102 glucose measurements at a given time interval (e.g. every minute).

At step 406 the patient 102, holding the smart insulin pen 106 (i.e. to enable the near-field communication link 112 with the patch 104), removes the pen cap 210 which serves as wake-up trigger. Alternatively, the wake-up trigger may be mechanical (e.g. pushing a button, turning a rotary knob, etc.).

The wake-up trigger is detected by the controller 208, which activates the pen 106 electronics and enables the NFEMI radio in step 408. The NFEMI in 408 side sends a wake-up request signal to the patch 104. In step 410, the NFEMI block in the patch 104 unit wakes-up and sends an acknowledge signal to the pen 106 to indicate the communication link is up-and-running.

In step 412 the controller 208 at pen 106 side now sends packetized data comprising of 1) pen ID, 2) medicine type, and 3) a remaining amount of insulin doses for the pen 106. The patch 104 side acknowledges that the data has been received.

Next in step 414, the patch 104 performs an authentication step which checks whether the received pen ID matches one of the stored pen IDs in the patch 104. The purpose of this authentication step is to ensure genuineness of the insulin pen 106 that is used by the patient 102. In case of no match, the pen 106 is not authenticated, the dose calculation process will not start and an indication is provided back to the pen 106 for stopping the process. In case of a match, the insulin pen 106 is authenticated and the dose calculation process will start. For the remainder we will assume this case.

In step 416, the patch 104 extracts the information received from the insulin pen 106. This information includes the type of insulin, and the remaining amount of insulin doses for the given pen 106. At a same time, the patch 104 unit retrieves the actual glucose reading (or alternatively glucose trend). In step 417, while accounting for type of insulin and glucose threshold levels, an insulin dose is calculated for the injection. At the same time, a check can be done whether the calculated insulin dosage does not exceed the available amount of insulin doses at pen 106 side.

In step 418, the patch 104 sends packetized data comprising of 1) patch ID, 2) a calculated required insulin dosage, and optionally 3) an indication when the needed dosage exceeds the available one. The insulin pen 106 side acknowledges that the data has been received.

Next in step 420, the insulin pen 106 performs an authentication step which checks whether the received patch ID matches one of the stored patch 104 ID at pen 106 side. The purpose of this authentication step is to ensure that the data is received from a genuine patch 104 unit that is worn by the patient 102. In case of no match, the patch 104 unit is not authenticated, the received information is ignored and the process will be stopped.

In step 422, in case of a match, the patch 104 unit is authenticated and the dose application process will start in step 424. The insulin pen 106 sets the calculated dose level at the Injector Level Control unit in step 426. In addition, the insulin pen 106 may display this information for the patient 102. Finally, the patient 102 needs to take action to doublecheck the dosage set value and modify when required. In step 428, the patient 102 inserts the needle, and take care of the insulin injection. The actual dosage is time-stamped, and the dosage log is updated accordingly.

Any potential process flow deadlock situations in this example 400 can be avoided using time-outs or wait-loops. Also note, that the order in which these instructions have been discussed does not limit the order in which other example embodiments implement the instructions unless otherwise specifically stated. Additionally, in some embodiments the instructions are implemented concurrently.

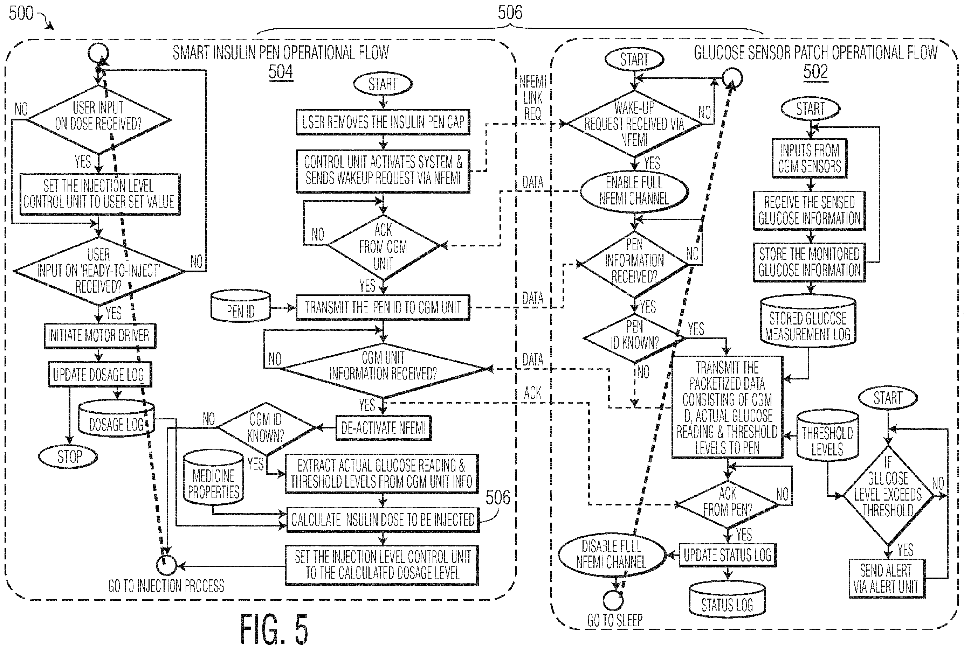

Figure 5 represents a second example 500 closed-loop operation 100 between the set of resource management devices (e.g. the patch 104 and the pen 106). The example 500 shows a glucose sensor patch 104 operational flow 502, a smart insulin pen 106 operational flow 504, and an authentication process 506 distributed between the patch 104 and the pen 106. As mentioned before, any potential process flow deadlock situations in this example 500 can be avoided using time-outs or wait-loops.

In this example 500, insulin dose calculation (i.e. step 506) is performed at the smart insulin pen 106 instead of at the patch 104 (i.e. step 417). This example 500 enables a different system partitioning with less computational burden at the patch 104 and more computational at the smart insulin pen 106. Otherwise the functionality as compared to example 400 in Figure 4 is substantially similar.

Any potential process flow deadlock situations in this example 500 can be avoided using time-outs or wait-loops. Also note, that the order in which these instructions have been discussed does not limit the order in which other example embodiments implement the instructions unless otherwise specifically stated. Additionally, in some embodiments the instructions are implemented concurrently.

In alternative embodiments of the closed-loop system 100 the near-field communication link 112 between patch 104 and smart insulin pen 106 may be replaced by the far-field communication link 114. In this case, Bluetooth Low Energy (BLE) can be used; however, far-field may not be ideal as it may suffer from body shadowing.

Alternative embodiments may also vary the authentication process may be considered such as only authenticating the patch ID but not the pen ID. In such a case, the patient 102 needs to ensure that a genuine smart insulin pen 106 is used. Most example embodiments however will always authenticate the patch ID to ensure detection of the 'right' set of glucose readings for the patient 102.

Some alternative embodiments may display the calculated dose setting only, and require the patient 102 to use this information for setting of the insulin motor driver. Also if a temperature sensor is available in the smart insulin pen 106, the pen 106 can prevent insulin injection if the temperature exceeds a threshold temperature.

One example discloses a resource management device, including: an input interface configured to receive a first set of sensor readings corresponding to a user and a second set of sensor readings corresponding to a set of resources; a controller configured to match the user, based on the first set of sensor readings, to a specific resource within the set of resources, based on the second set of sensor readings; wherein the controller is configured to authenticate that the first set of sensor readings originated from the user before the match.

The instructions and/or flowchart steps in the above Figures can be executed in any order, unless a specific order is explicitly stated. Also, those skilled in the art will recognize that while one example set of instructions/method has been discussed, the material in this specification can be combined in a variety of ways to yield other examples as well, and are to be understood within a context provided by this detailed description.

In some example embodiments the set of instructions described above are implemented as functional and software instructions. In other embodiments, the instructions can be implemented either using logic gates, application specific chips, firmware, as well as other hardware forms.

When the instructions are embodied as a set of executable instructions in a non-transitory computer-readable or computer-usable media which are effected on a computer or machine programmed with and controlled by said executable instructions. Said instructions are loaded for execution on a processor (such as one or more CPUs). Said processor includes microprocessors, microcontrollers, processor modules or subsystems (including one or more microprocessors or microcontrollers), or other control or computing devices. A processor can refer to a single component or to plural components. Said computer-readable or computer-usable storage medium or media is (are) considered to be part of an article (or article of manufacture). An article or article of manufacture can refer to any manufactured single component or multiple components. The non-transitory machine or computer-usable media or mediums as defined herein excludes signals, but such media or mediums may be capable of receiving and processing information from signals and/or other transitory mediums.

Example embodiments of the material discussed in this specification can be implemented in whole or in part through network, computer, or data based devices and/or services. These may include cloud, internet, intranet, mobile, desktop, processor, look-up table, microcontroller, consumer equipment, infrastructure, or other enabling devices and services. As may be used herein and in the claims, the following non-exclusive definitions are provided.

It will be readily understood that the components of the embodiments as generally described herein and illustrated in the appended figures could be arranged and designed in a wide variety of different configurations. Thus, the detailed description of various embodiments, as represented in the figures, is not intended to limit the scope of the present disclosure, but is merely representative of various embodiments. While the various aspects of the embodiments are presented in drawings, the drawings are not necessarily drawn to scale unless specifically indicated.

The present invention may be embodied in other specific forms without departing from its spirit or essential characteristics. The described embodiments are to be considered in all respects only as illustrative and not restrictive. The scope of the invention is, therefore, indicated by the appended claims rather than by this detailed description. All changes which come within the meaning and range of equivalency of the claims are to be embraced within their scope.

Reference throughout this specification to features, advantages, or similar language does not imply that all of the features and advantages that may be realized with the present invention should be or are in any single embodiment of the invention. Rather, language referring to the features and advantages is understood to mean that a specific feature, advantage, or characteristic described in connection with an embodiment is included in at least one embodiment of the present invention. Thus, discussions of the features and advantages, and similar language, throughout this specification may, but do not necessarily, refer to the same embodiment.

Furthermore, the described features, advantages, and characteristics of the invention may be combined in any suitable manner in one or more embodiments. One skilled in the relevant art will recognize, in light of the description herein, that the invention can be practiced without one or more of the specific features or advantages of a particular embodiment. In other instances, additional features and advantages may be recognized in certain embodiments that may not be present in all embodiments of the invention.

Reference throughout this specification to "one embodiment," "an embodiment," or similar language means that a particular feature, structure, or characteristic described in connection with the indicated embodiment is included in at least one embodiment of the present invention. Thus, the phrases "in one embodiment," "in an embodiment," and similar language throughout this specification may, but do not necessarily, all refer to the same embodiment.

## Claims

1. A resource management device, comprising:
an input interface configured to receive a first set of sensor readings corresponding to a user and a second set of sensor readings corresponding to a set of resources;
a controller configured to match the user, based on the first set of sensor readings, to a specific resource within the set of resources, based on the second set of sensor readings;
wherein the controller is configured to authenticate that the first set of sensor readings originated from the user before the match.

2. The device of claim 1:
wherein the first set of sensor readings correspond to a set of glucose readings;
wherein the set of resources include insulin; and
wherein the second set of sensor readings correspond to an attribute of the insulin.

3. The device of any preceding claim :
wherein the controller is configured to authenticate the set of resources correspond to the second set of sensor readings before the match.

4. The device of any preceding claim:
wherein the input interface is a near-field wireless communications interface.

5. The device of claim 4:
wherein the first set of sensor readings are generated from the user via a patch coupled to the user; and
wherein the near-field interface is enabled only when the user is holding or touching the specific resource.

6. The device of claim 5:
wherein the patch is a glucose sensor patch; and
wherein the specific resource is an insulin pen.

7. The device of any of claims 4 to 6:
wherein the first set of sensor readings are generated from the user via a bio-identity sensor coupled to identify the user; and
wherein the near-field interface is enabled only when the user is touching the specific resource.

8. The device of any preceding claim:
wherein the controller is configured to wake-up the resource management device from a low power state in response to a trigger event from the user.

9. The device of claim 8:
wherein the specific resource is an insulin pen; and
wherein the trigger event corresponds to the user removing a pen cap from the insulin pen.

10. The device of any preceding claim:
further comprising an authentication unit configured to enable the controller to authenticate by verifying at least one of: a unique identifier (ID), a manufacturing certificate, a remaining amount of the specific resource, an expiration of the specific resource, an environmental history of the specific resource, and/or a type of the specific resource.

11. The device of any preceding claim:
wherein the specific resource is an insulin pen; and
further comprising an authentication unit configured to enable the controller to authenticate by verifying at least one of: a unique identifier (ID) of the insulin pen, a manufacturing certificate for the insulin pen, a remaining amount of insulin in the insulin pen, an expiration date of the insulin in the insulin pen, an environmental history of the insulin in the insulin pen, and/or a type of insulin in the insulin pen.

12. The device of any preceding claim:
wherein the first set of sensor readings correspond to a set of glucose readings from a patch coupled to the user; and
further comprising an authentication unit configured to enable the controller to authenticate by verifying at least one of: a unique identifier (ID) of the patch, a manufacturing certificate for the patch, a glucose measurement log in the patch, and/or an insulin dosage history in the patch.

13. The device of any preceding claim:
wherein the first set of sensor readings correspond to a set of glucose readings from a patch coupled to the user; and
further comprising an alerting unit configured to enable the controller to alert the user when the user needs another insulin injection within a predefined time window.

14. The device of any preceding claim:
wherein the specific resource is insulin;
wherein the first set of sensor readings correspond to a set of glucose readings from a patch coupled to the user; and
further comprising an alerting unit configured to enable the controller to alert the user when the specific resource contains an insufficient amount or incorrect type of the insulin needed for injection by the user.

15. A method of distributing instructions, stored on a non-transitory, tangible computer readable storage medium, for configuring a resource management device:
wherein the device includes an input interface and a controller; and
wherein the instructions include,
configuring the input interface to receive a first set of sensor readings corresponding to a user and a second set of sensor readings corresponding to a set of resources;
configuring the controller to match the user, based on the first set of sensor readings, to a specific resource within the set of resources, based on the second set of sensor readings; and
wherein the controller is configured to authenticate the user and the specific resource before the match.
